(19) Europäisches Patentamt
European Patent Office
Office européen des brevets

(11) **EP 1 629 817 A1**

(12) **EUROPEAN PATENT APPLICATION**

(43) Date of publication:
**01.03.2006 Bulletin 2006/09**

(51) Int Cl.:
*A61H 1/00* (2006.01)      *A61H 37/00* (2006.01)
*A61B 5/053* (2006.01)

(21) Application number: **05018707.9**

(22) Date of filing: **29.08.2005**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **30.08.2004 JP 2004250655**

(71) Applicant: **Omron Healthcare Co., Ltd.
Kyoto 615-0084 (JP)**

(72) Inventor: **Kasai, Eiji
c/o KETEC CO., Ltd.
Kyoto 617-0002 (JP)**

(74) Representative: **Wilhelms . Kilian & Partner
Patentanwälte
Eduard-Schmid-Strasse 2
81541 München (DE)**

(54) **Massager**

(57)      A massager includes a back rest portion, massage heads (402a-402d), a treatment section (40), a sensor section (100), and a control section (13). The massage heads are provided to the back rest portion, and massage a user. The treatment section moves a pair of massage head arms (403R,403L) supporting the massage heads, in association with a massage action. A sensor section (113) senses a bio-signal of the user from an impedance variance between the pair of massage head arms. The control section controls the massage action of massage heads, causes the treatment section to move, and detects a signal corresponding to the bio-signal in a predetermined range, thereby determine a physical constitution of the user.

FIG. 1

**Description**

Background of the invention

Field of the invention

The present invention relates to a massager. More specifically, the invention relates to a massager that uses a sensor for sensing a physical constitution of a user.

Description of the related art

In many conventional massagers, a massage action is performed in accordance with a shoulder position of a user. In addition, there are massagers of the type that performs start/stop action in accordance with the presence or absence of the sitting posture of a user.

Means of the type determining the shoulder position in the massager and the presence or absence of the sitting posture of the user include those described herebelow.

Japanese Patent No. 3128260 discloses a massager that sensing a massage site of the shoulders of a user in such a manner that a strain gauge is used to detect the pressure of a roller onto the massage site or to detect the angle of an arm provided with the roller.

Japanese Unexamined Patent Application Publication No. 2003-102799 discloses a massager using a plurality of infrared-light receiving photosensors deployed at heights different from one another to a front face of a back rest. In this case, the shoulder position of a user is detected from the position of the photosensors blocked off by the user from an infrared ray emitted from a remote control unit.

Japanese Unexamined Patent Application Publication No. 11-42263 discloses a massager that detects the presence or absence of a person and detects the shoulder position of the person in accordance with electrostatic capacitance detected by a plurality of detectors provided to a treatment frame assembly.

However, with any of the massagers disclosed in the above-referenced patent publications, while the shoulder position of the user can be substantially sensed, the physical constitution of the user cannot be sensed.

Summary of the invention

The present invention is made in view of the related art described above. Accordingly, an object of the present invention is to provide a massager that has a simple structure, and accurately senses the physical constitution of a user.

Another object of the present invention is to provide a massager capable of controlling a massage action in correspondence to the physical constitution of the user.

To achieve the above-mentioned object, the present invention provides a massager comprising:

a back rest portion that supports a back side of a user;
treatment applicators that are provided to the back rest portion and that massage the user;
a treatment section that moves a pair of electrodes supporting the treatment applicators, in association with a massage action;
a bio-signal sensor that senses a bio-signal of the user from an impedance variance between the pair of electrodes; and
a control section that controls the massage action of the treatment applicators in accordance with the bio-signal, wherein
the bio-signal sensor includes:

an oscillation section that supplies a high frequency signal;
the pair of electrodes that are disposed to allow the user to near or contact and that are provided to receive the high frequency signal from the oscillator section;
a sensor section that senses the impedance variance occurring between the electrodes having received the high frequency signal when the user has neared and contacted the electrodes; and
a signal processor section that processes a signal corresponding to the sensed impedance variance into a desired signal corresponding to the bio-signal,
wherein the control section causes the treatment section to move and detects a signal corresponding to the bio-signal in a predetermined range, thereby to determine a physical constitution of the user.

Further, according to another aspect of the present invention, there is provided a massager comprising:

a back rest portion that supports a back side of a user;

treatment applicators that are provided to the back rest portion and that massage the user;

a treatment section that moves a pair of electrodes supporting the treatment applicators, in association with a massage action;

a bio-signal sensor that senses a bio-signal of the user from an impedance variance between the pair of electrodes; and

a control section that controls the massage action of the treatment applicators in accordance with the bio-signal, wherein

the bio-signal sensor includes:

an oscillation section that supplies a high frequency signal;

the pair of electrodes that are disposed to allow the user to near or contact and that are provided to receive the high frequency signal from the oscillator section;

a sensor section that senses the impedance variance occurring between the electrodes having received the high frequency signal when the user has neared and contacted the electrodes; and

a signal processor section that processes a signal corresponding to the sensed impedance variance into a desired signal corresponding to the bio-signal,

wherein the control section causes the treatment section to move and performs averaging of a plurality of signals corresponding to the bio-signal in a predetermined range, thereby to determine a physical constitution of the user.

The terminology "physical constitution" of the user is herein used to refer to not only an exterior body form of the user, but also is used in a broader sense to refer a somatic part of the user, including distributions of fat and muscles. As such, even in the case that exterior body forms are identical in type to one another, a different signal is generated depending on whether a person (user) is of a type rich in muscle or of a type rich in fat.

According to these configurations, without requiring a user to set the user's own physical constitution, a massage action corresponding to the physical constitution of the user can be automatically selected. Consequently, a comfortable massage action can be performed without imposing, for example, excessive burden and uncomfortable feeling on the user.

Preferably, there is provided a massager, wherein the control section makes a comparison between an average value obtained through the averaging of the plurality of signals and a predetermined reference value, thereby to determine the physical constitution of the user.

According to the configuration, error attributed signal distortion can be reduced, consequently enabling high accuracy sensing of the physical constitution.

Preferably, there is provided a massager, comprising a seat portion whereon the user takes a sitting posture, wherein the predetermined range is defined such that a portion near an upper surface of the seat portion is preset to be a lower end, and a portion near a predetermined site of a body of the user in a use state of the massager is preset to be an upper end.

Preferably, there is provided a massager, wherein the predetermined range is a range below a position of a predetermined site of a user body determined from the bio-signal of the user, and is set corresponding to the user.

According to these configurations, the physical constitution of the user is determined in a predetermined range of, for example, from a shoulder blade to a waist portion, where signal variations due to the body length are relatively small. This enables high accuracy determination of the physical constitution of the user.

Preferably, there is provided a massager, wherein the control section changes a pattern of massage being performed with the treatment applicators in accordance with the physical constitution of the user having been determined.

Preferably, there is provided a massager, wherein the control section changes a pressure force being applied with the treatment applicators in accordance with the physical constitution of the user having been determined.

According to these configurations, since the massage action is changeable corresponding to the physical constitution of the user, the appropriate massage actions can be performed even for users having physical constitutions different in the type from one another.

Preferably, there is provided a massager, wherein the electrodes are pivotable with respect to the treatment section, and are arms each supporting a plurality of the treatment applicators; and the plurality of treatment applicators are provided to be aligned substantially parallel to a movement direction of the treatment section in a manner to sandwich a pivoting center of the arm.

According to the configuration, among the plurality of treatment applicators supported to the respective arm, when one of the treatment applicators provided on the side of the moving direction of the treatment section abuts the user, reactive forces from the user act on the abutted one treatment applicator, thereby to pivotally move the arm. Thereby, the one treatment applicator and the other treatment applicator, which is provided opposite the one treatment applicator, move closer to the user. Consequently, the bio-signal from the user can be accurately sensed, thereby making it possible to determine the position of an uncomfortable site of the user even more accurately.

Preferably, there is provided a massager, wherein the sensor section generates and supply a signal that represents a variance in a level of a reflected wave of the high frequency signal and that corresponds to the impedance variance occurring between the electrodes in correspondence to a physical constitution of the user.

Preferably, there is provided a massager, wherein the sensor section outputs reflection wave signals corresponding to real and imaginary impedance variances occurring between the electrodes in correspondence to a physical constitution of the user.

Preferably, there is provided a massager, wherein the frequency of the high frequency signal is in a range of from 10 MHz to 300 MHz.

According to the present invention, it is possible to realize a massager that has a simple structure, and accurately senses the physical constitution of the user.

Brief description of the drawings

FIG. 1 is a schematic block diagram showing a sensor section included in an electrical configuration of a massager according to an embodiment of the present invention;

FIG. 2 is an overall front elevation view showing schematic configuration of the massager according to the embodiment;

FIG. 3 is an overall side view showing schematic configuration of the massager according to the embodiment;

FIG. 4 is a front elevation view of elevating treatment unit according to the embodiment;

FIG. 5 is rearside elevation view of the elevating treatment unit according to the embodiment;

FIG. 6 is a perspective view of a treatment section of the massager according to the embodiment;

FIG. 7 is a schematic block diagram of an electrical configuration of a massager according to the embodiment;

FIG. 8 is a graph showing an example of variances in output obtained from the sensor section in the massager according to the embodiment;

FIG. 9 is a flow diagram showing operation of the massager, according to a first embodiment example;

FIG. 10 is a flow diagram showing operation of the massager, according to a second embodiment example;

FIG. 11 is a flow diagram showing operation of the massager, according to a third embodiment example;

Detailed description of the preferred embodiments

With reference to the accompanying drawings, the preferred embodiments for carrying out the present invention will be described in detail hereinbelow. However, unless specifically described, it is not intended that the scope of the present invention is not limited to dimensions, materials, shapes, and functions of components, and relative deployment of the components described below in the embodiments. In addition, unless newly described, materials, shapes, functions, and the like of members initially described remains similar or identical for those subsequently described.

(Overall Configuration of Massager)

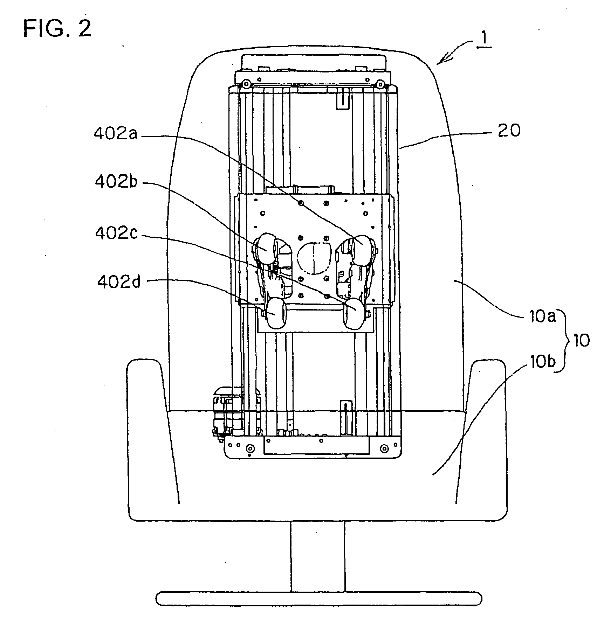

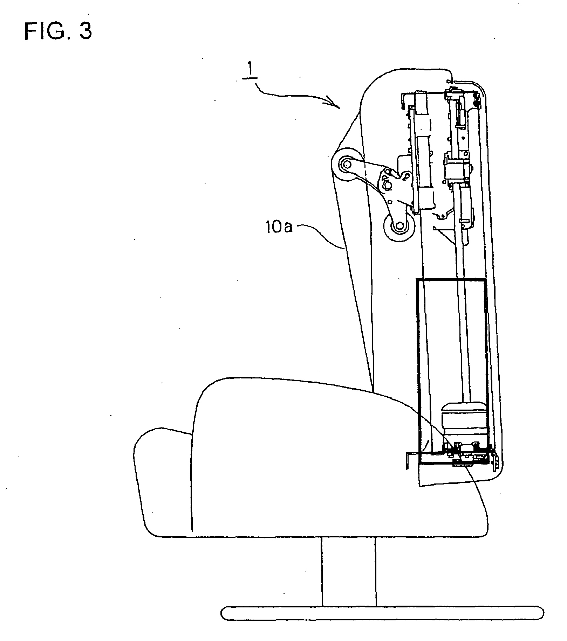

FIG. 2 is an overall front elevation view showing a schematic configuration of a massager 1 capable of preferably carrying out the present invention. FIG. 3 is an overall side view showing a schematic configuration of the massager 1, which is capable of preferably carrying out the present invention.

The massager 1 includes a reclinable chair 10 and an elevating treatment unit 20. The chair 10 consist of a back rest portion 10a for supporting the back face of a user, and a seat portion 10b, wherein elevating treatment unit 20 is assembled into the back rest portion 10a.

The elevating treatment unit 20 includes massage heads 402a to 402d (treatment applicators) provided to protrude toward the side of a surface covered by a cover sheet of the back rest portion 10a. Human body massaging is done by the massage head 402a to 402d.

The massage heads 402a to 402d are provided to the back rest portion 10a, thereby to massage the user. More specifically, the massage heads 402a to 402d are separated into the massage heads 402a and 402b (.first treatment applicators) provided in a pair in upper right and left portions along the spine direction, and the massage heads 402c and 402d (second treatment applicators) provided in a pair in lower right and left portions below the massage heads 402a and 402b.

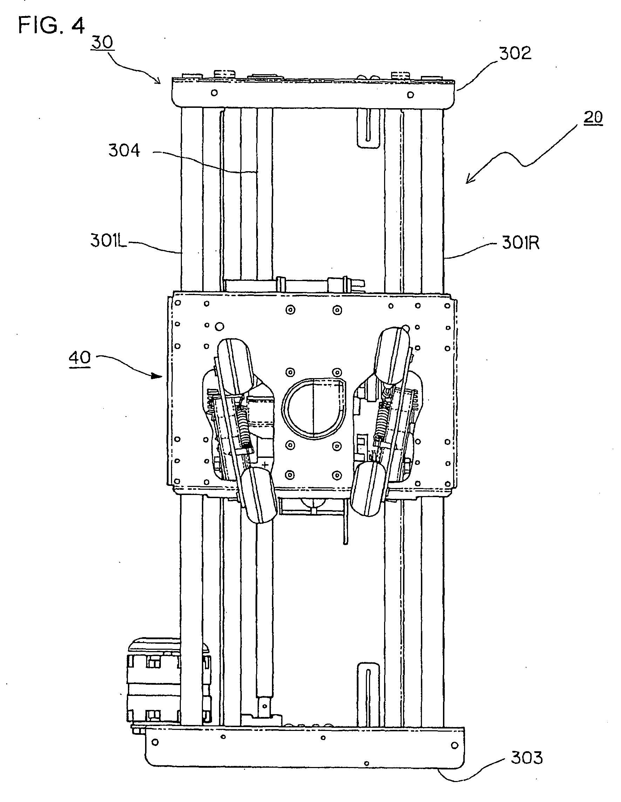

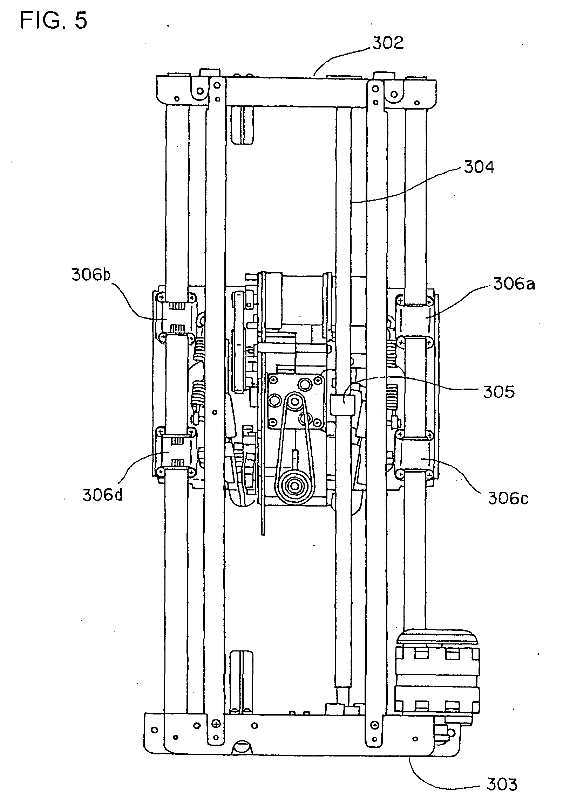

FIG. 4 is a front elevation view of the elevating treatment unit. FIG. 5 is a rearside elevation view of the elevating treatment unit.

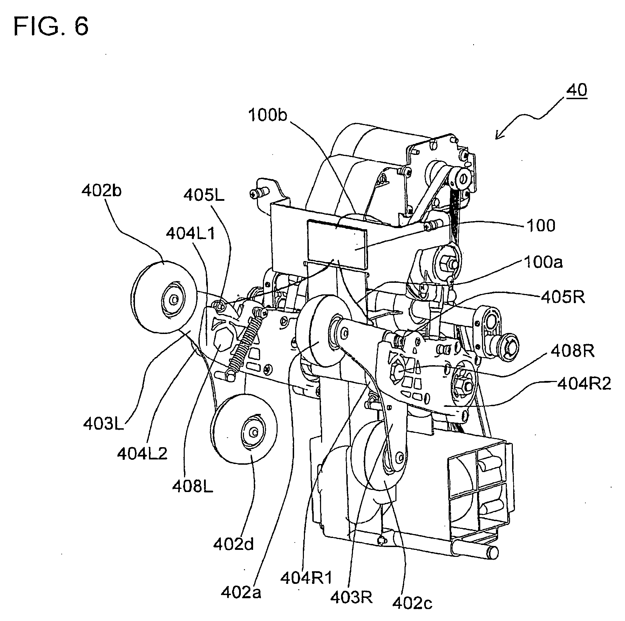

The elevating treatment unit 20 includes an elevation section 30 and a treatment section 40 and thereby to move the arms 403R and 403L in association with the massage action. The arms 403R and 403L functions as a pair of electrodes that support the treatment applicators (refer to FIG. 6) . With reference to in FIG. 4, the elevation section 30 includes a pair of guide pipes 301R and 301L that are each circular in cross section and are disposed along the back rest portion 10a; elevation-dedicated threaded shaft 304 disposed parallel to and between the two guide pipes 301R and 301L; and a pair of upper and lower guide pipe holders 302 and 303 disposed perpendicular to the guide pipes 301R and 301L.

The guide pipes 301R and 301L are secured to guide pipe holders 302 and 303. The elevation-dedicated threaded shaft 304 is rotatably supported.

With reference to FIG. 5, the treatment section 40 is supported by elevation guides 306a, 306b, 306c, 306d supported axially movably to the guide pipes 301R and 301L and by an elevation nut holder (not shown) that holds an elevation nut 305 engaging an outer circumference of the elevation-dedicated threaded shaft 304.

In conjunction with the elevation nut 305 being vertically driven by the rotation of the elevation-dedicated threaded shaft 304, the elevation nut holder and the treatment section 40 supporting the elevation nut holder are vertically moved along the guide pipes 301R and 301L.

(Schematic Configuration of Treatment Section)

The schematic configuration of the treatment section 40 will be described hereinbelow. FIG. 6 is a perspective view of the treatment section 40 of the massager 1 capable of preferably carrying out the present invention.

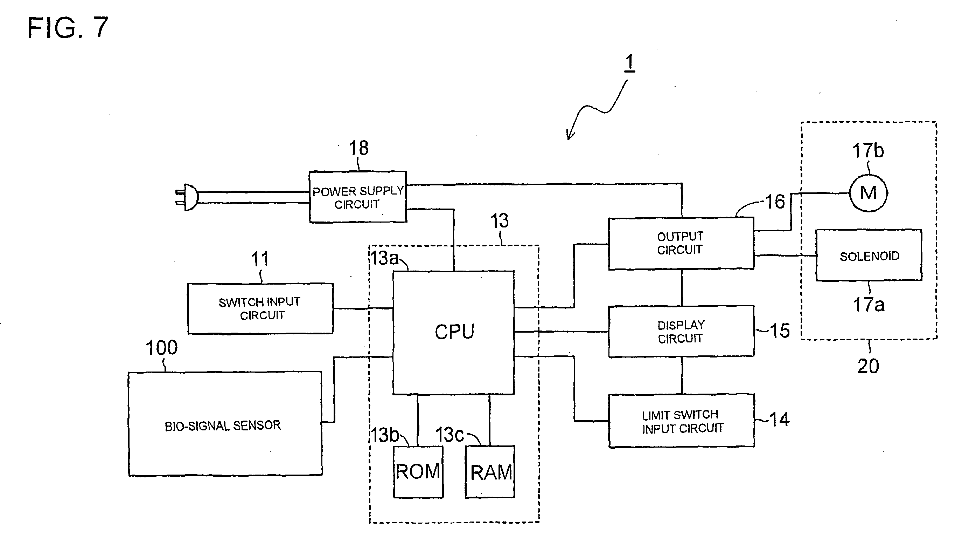

The treatment section 40 has the four massage heads 402a to 402d, the massage head arms 403R and 403L which is substantially "V" shape, a bio-signal sensor 100 (hereinafter referred to as "sensor section"), and a control section 13 (refer to FIG. 7). The sensor section 100 oscillates a signal of a predetermined frequency, and senses a bio-signal emitted from the user from an impedance variance between the massage head arms 403R and 403L. The control section 13 controls the massage action of the massager 1 including the treatment section 40 in accordance with the bio-signal output from the sensor section 100.

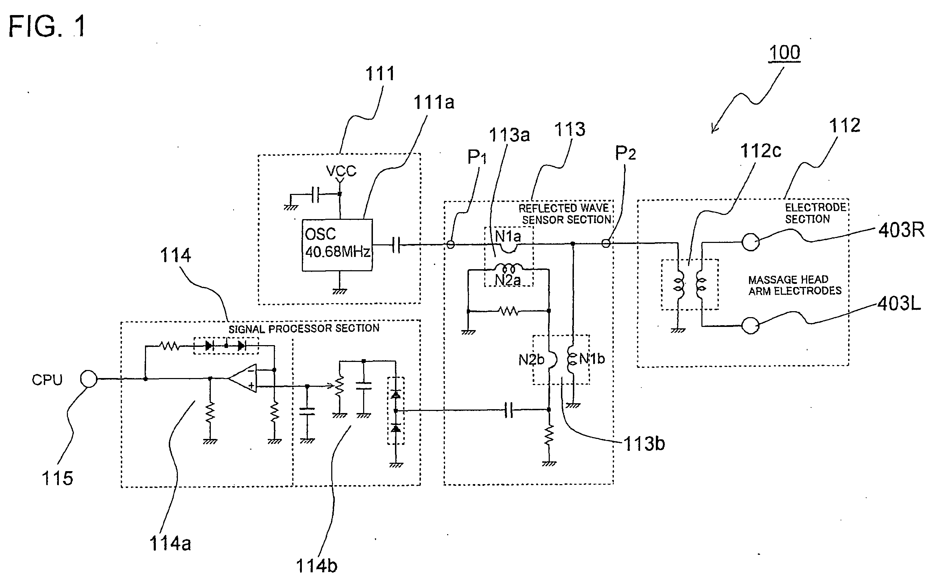

The massage head arms 403R and 403L respectively function as electrodes for sensing the shape and presence or absence of a part of the human body. The arms 403R and 403L are connected to a reflected wave sensor section 113 (refer to FIG. 1) of the sensor section 100 through a pair of power feed lines 100a provided to supply high frequency signals from an oscillator section 111 (refer to FIG. 1) . Ends of the power feed line 100a are connected by, being screwed or soldered, to the respective massage head arms 403R and 403L.

The massage head arms 403R and 403L have end portions respectively rotatably journaling two of the massage heads 402a to 402d.

A base end portion of the massage head arm 403R is sandwiched by members 404R1 and 404R2 of an arm support member 404R, and is supported by a pivotal shaft 408R. A base end portion of the massage head arm 403L is sandwiched by members 404L1 and 404L2 of the arm support member 404L, and is supported by a pivotal shaft 408L. Thereby, the massage head arms 403R and 403L are rotatable with respect to the treatment section 40.

The plurality of massage heads 402a to 402d provided to the respective massage head arms 403R and 403L are provided substantially parallel to the movement direction of the treatment section 40.

In addition, stoppers 405R and 405L for stopping the pivotal movements are provided to the massage head arms 403R and 403L, respectively.

The massage head arms 403R and 403L are each pivotally movable with respect to the treatment section 40, and each support plural ones of massage heads 402a to 402d. The plural ones of the massage heads 402a to 402d are preferably aligned and provided substantially parallel to the movement direction of the treatment section 40 with a pivoting center of each of the massage head arm 403R and 403L being interposed.

According to the configuration, among the plurality of massage heads 402a to 402d supported to the massage head arms 403R and 403L, when the one-side massage heads 402c and 402d provided on the side of the moving direction of the treatment section 40 abut a user, reactive forces from the user act on the abutted one-side massage heads 402c and 402d, thereby to pivotally move the massage head arms 403R and 403L. Thereby, the one-side massage heads 402c and 402d and the other-side massage heads 402a and 402b provided on the opposite side move even closer to the user, consequently making it possible to accurately sense the bio-signal from the user.

(Control circuit of Massager)

FIG. 7 is a schematic block diagram showing an electrical configuration of the massager 1 according to the present invention.

With reference to FIG. 7, the massager 1 includes, mainly, a switch input circuit 11, the sensor section (bio-signal sensor) 100, the control section 13 for controlling the operation of the massager 1, a limit switch input circuit 14, a display circuit 15, an output circuit 16, the elevating treatment unit 20, and a power supply circuit 18.

The control section 13 includes a central processing unit (CPU) 13a, a read-only memory (ROM) 13b, and a random access memory (RAM) 13c. The elevating treatment unit 20 is provided to the back rest portion 10a, and includes a solenoid 17a and a motor 17b.

Although not shown, in addition to a power switch, the switch input circuit 11 includes key switches for specifying various motions of elevating treatment unit 20, such as, for example, kneading (massaging), patting, ascension, and descension.

The sensor section 100 is a bio-signal sensor for sensing bio-signals of the user, and generates a bio-signal of, for example, the frequency of pulses or the frequency of respirations, thereby to supply the signal to the CPU 13a of the control section 13. Such a bio-signal is variable depending on the difference in the user's body type, muscles, fat, or the like, and is used such that the body type, body fat percentage, and the like of the user is indirectly measured and computed through the signal processing of the bio-signal.

In the event of using the massager 1, when the power switch (not shown) of a switch input circuit 11 is turned on by a user, CPU 13a responsively activates the power supply circuit 18, and the power supply circuit 18 in turn supplies power to respective sections of the massager 1.

In response to a specification given by the user through the switch input circuit 11, the CPU 13a generates a signal for driving the elevating treatment unit 20 and supplies the signal to the output circuit 16. In response to the signal, the output circuit 16 drives the solenoid 17a and the motor 17b, which constitute the elevating treatment unit 20, thereby to implement a motion specified by the user.

In addition, the CPU 13a drives the display circuit 15, and displays necessary information such as contents specified by the user to be viewable by the user.

The limit switch input circuit 14 defines a range of the vertical movement of the treatment section 40 in the elevating treatment unit 20 on the back rest portion 10a. In the event that the treatment section 40 ascends to reach the position of an upper limit or descends to reach the position of a lower limit, a limit switch is driven, thereby to stop the vertical movement of the treatment section 40.

The overall action of the massager 1 shown in FIG. 2 in accordance with the bio-signal sensed by the treatment section 40 according to the present invention will be described below.

(Circuit Configuration of Sensor Section)

FIG. 1 is a schematic block diagram showing a sensor section included in an electrical configuration of the massager 1 shown in FIG. 7.

As shown in FIG. 1, the sensor section 100 includes the oscillator section 111, an electrode section 112, the reflected wave sensor section 113, a signal processor section 114, and an output terminal 115. More specifically, the electrode section 112 includes the massage head arms 403R and 403L, which functions as electrodes, and a transformer 112c.

The pair of massage head arms 403R and 403L is disposed so as to enable a user to near or contact, is provided to receive high frequency signals from the oscillator section 111, and is connected to the treatment section 40.

Operation of the sensor section 100 will be outlined herebelow. The oscillator section 111 supplies a high frequency signal, and the massage head arms 403R and 403L receives the high frequency signal therefrom through the transformer 112c being used for isolation and real value conversion. In more specific, both ends of a primary coil of a transformer 112c are, respectively, coupled to the reflected wave sensor section 113 and the ground potential, and both ends of a secondary coil are, respectively, connected to the massage head arms 403R and 403L.

As described below, the impedances in the massage head arms 403R and 403L are fluctuated depending on the human body movement, shape, and the like. As such, also the energy of the high frequency signal being consumed at the electrodes is fluctuated depending on the human body movement, shape, and the like. Accordingly, a variance in energy of the high frequency signal remained unconsumed at the electrode is sensed as a variance in reflection level by the reflected wave sensor section 113. The sensed variance in the reflection wave level is amplified and signal-processed by the signal processor section 114, and is then output in the form of a bio-signal from the output terminal 115. The output bio-signal is supplied to the CPU 13a, which controls the massager 1 (refer to FIG. 7).

As shown in FIG. 2, the massage head arms 403R and 403L are disposed on the reverse side of the surfacemember of the back rest portion 10a of the massager 1, and the sensor section 100 integrally configured of other circuit elements is disposed in the treatment section 40 provided inside of the back rest portion 10a (refer to FIG. 6). The aforementioned other circuit elements are, as shown in FIG. 1, the transformer 112c, oscillator section 111, reflected wave sensor section 113, and signal processor section 114. Although not shown, the bio-signal output from the output terminal 115 of the sensor section 100 is supplied to the CPU 13a (refer to FIG. 7) of the control section 13 disposed inside of the massager 1.

A configuration of the oscillator section 111 of the sensor section 100 shown in FIG. 1 will now be described herebelow.

The oscillator section 111 has an oscillator 111a, such as a crystal oscillator, thereby constituting an oscillator circuit for outputting high frequency pulse signals. The oscillator section 111 further has two lowpass filters (not shown) for transforming pulse signals into sinusoidal waves, and an attenuator (not shown).

Such a configuration is made for the reason that, only in the event a sinusoidal wave signal is output as a high frequency output of the oscillator section 111, when an impedance match is recognized between the oscillator section 111 and the massage head arm 403R, 403L, the overall energy of a sinusoidal wave component is consumed at the electrode to cause the level of the reflected wave from the electrode to be zero. In other words, unless the output is a sinusoidal wave signal, energy of a non-sinusoidal wave component is reflected from an all-time loaded electrode, regardless of impedance matching.

For the high frequency component, the frequency thereof is appropriately selectable, but the frequency is preferably within a range of from 10 MHz to 300 MHz. In the present embodiment, the frequency is set to 40.68 MHz, but is not limited thereto. For example, 13.56 MHz and 27.12 MHz are preferable frequencies as well.

A configuration of the reflected wave sensor section 113 of the sensor section 100 shown in FIG. 1 will be described herebelow.

The reflected wave sensor section 113 has terminals $P_1$ and $P_2$ and M coupled circuits 113a and 113b each having a well-known configuration. An oscillation output of the oscillator section 111 is coupled to the terminal $P_1$, an input end of the electrode section 112 (one end of the primary coil of the transformer 112c) is coupled to the terminal $P_2$.

The M coupled circuit 113a is configured of a primary coil N1a inserted between the terminals $P_1$ and $P_2$, a secondary coil N2a, and a resistor R parallel connected to both ends of the secondary coil N2a. The M coupled circuit 113b is configured of a primary coil N1b inserted between the terminal $P_2$ and a ground potential, and a secondary coil N2b. The secondary coils N2a and N2b are each series connected to the ground potential and an output end of the reflected wave sensor section 113.

Principles for sensing of the bio-signal by the reflected wave sensor section 113 will be described in detail herebelow.

By way of example, it is assumed that in the chair type massager 1 shown in FIG. 2, a user is taking a correct sitting posture along the back rest portion 10a. More specifically, it is assumed that the user is taking the correct sitting posture so that the shoulder position of the user is positioned near the massage head arm 403R, 403L.

When the high frequency sinusoidal wave signal supplied from the oscillator section 111 is supplied to the electrodes, a flow of a high frequency wave current occurs between the two massage head arms 403R and 403L through the surface member of the massager 1, the clothing of the user, and human body tissue (primarily, a fat component) of the user.

In more exact words, when the user nears or contacts the massage head arms 403R and 403L, the reflected wave sensor section 113 senses an impedance variance occurring between the massage head arms 403R and 403L functioning as the electrode receiving the high frequency signal.

The factor such as a distance variance between an electrode surface and a human body surface depending on the human body movement or a distance variance from the respective electrode surface to the human body tissue depending on the human body type corresponds to a variance in an imaginary component of a high frequency impedance in the electrode. In addition, the deformation in the human body tissue depending on the human body movement corresponds to a variance in a real component of the high frequency impedance.

The former distance variance and the latter human body tissue variance cyclically occur depending on the shape on the back side of the user who is taking a sitting posture. Consequently, a high frequency impedance variance corresponding to the human body movement or human physical constitution of the user occurs at the respective electrode.

In other words, the reflected wave sensor section 113 generates and supplies a signal that represents a variance in the level of the reflected wave of the high frequency signal and that corresponds to the impedance variance occurring between the massage head arms 403R and 403L in correspondence to the physical constitution of the user.

Alternatively, the reflected wave sensor section 113 may be configured to output reflection wave signals corresponding to real and imaginary impedance variances occurring between the massage head arms 403R and 403L in correspondence to the physical constitution of the user.

In general, an impedance Z is expressed as follows:

$$Z = R + j(\omega L - (1/\omega C)) \qquad (equation\ 1)$$

where,

R: resistance;
L: inductance;
C: capacitance; and

ω: angular frequency

The high frequency signal supplied from the oscillator section 111 is all consumed at the massage head arms 403R and 403L if the impedance match is attained between the massage head arms 403R and 403L. However, the energy consumed at the electrodes is varied corresponding to the impedance variance between the electrodes, and residual energy is returned as a reflected wave from the electrode section 112 to the oscillator section 111 through the reflected wave sensor section 113. The M coupled circuits 113a and 113b of the reflected wave sensor section 113 form directional couplers, whereby a part of energy unconsumed at the electrodes is taken out and supplied as a reflected wave output to the signal processor section 114 at a rear stage.

The M coupled circuit using the coil is just a well-known example of directional coupler, and the reflected wave sensor section 113 can be alternately configured by using, for example, capacitors and microstrip lines.

A configuration of the signal processor section 114 of the sensor section 100 shown in FIG. 1 will be described hereinbelow.

The signal processor section 114 has an amplifier 114a and a filter 114b. As described above, a signal indicative of the variance in the level of the reflected wave at the massage head arms 403R and 403L is supplied to the signal processor section 114 from the reflected wave sensor section 113. The signal is then amplified in the signal processor section 114 and is passed through the filter wherein an appropriate constant is set, whereby a signal corresponding to the physical constitution of the user can be output.

In other words, the signal processor section 114 processes the signal corresponding to the sensed impedance variance into a signal corresponding to a desired bio-signal.

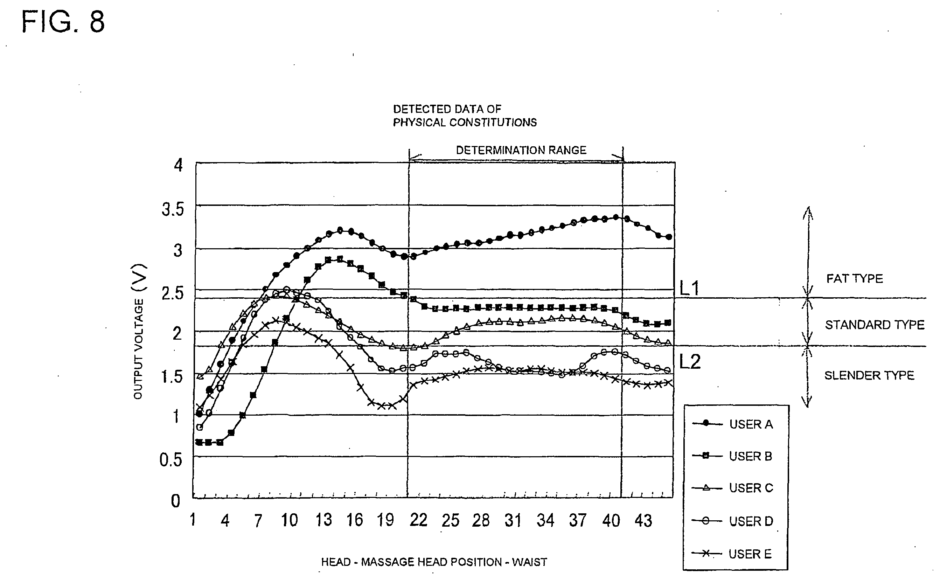

The treatment section 40, which has the massage head arms 403R and 403L, is driven by the elevating treatment unit 20 to descend from the upper portion to the lower portion of the back rest portion 10a in correspondence to the number of pulses of the motor. In the present embodiment, the signal is sensed on the basis of the number of elevation pulses of the motor, and a variance graph of the signal output voltage shown in FIG. 8 is obtained.

The sensed signal is serially supplied to the control section 13 of the massager 1, and the physical constitution of the user is determined in the CPU 13a. In accordance with the information, the massage action is controlled.

The following provides description of measurement for sensing the physical constitution of the user and control of the massage action that are carried out in the massager 1 according to the above-described embodiment. The description is made with reference to respective embodiment examples.

[First Embodiment Example]

(Sensing Method for Physical Constitution)

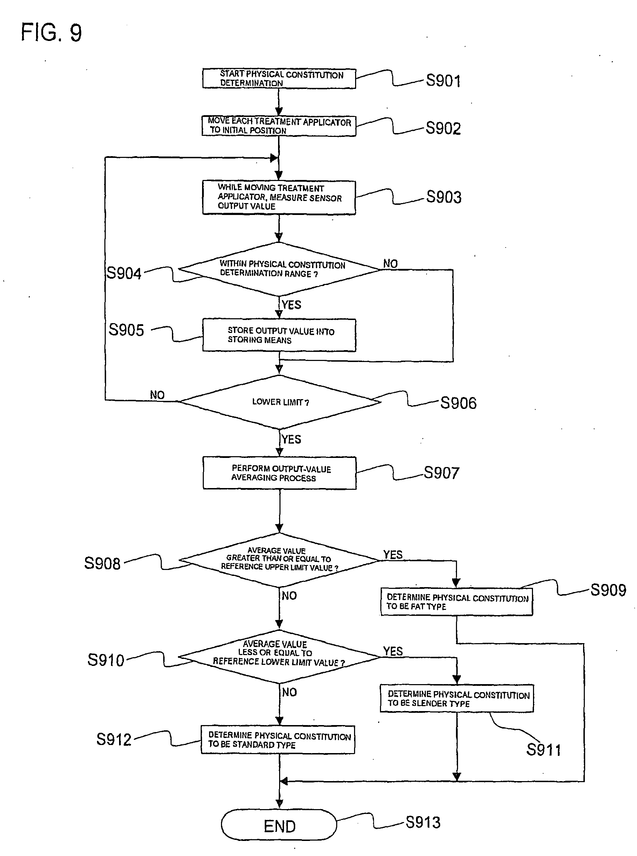

FIG. 9 is a flow diagram in the event of sensing the physical constitution of a user in the massager 1.

In the massager 1 according to the embodiment, in the state where the massage action is not performed, the treatment section 40 is placed in a standby state in an upper portion of the back rest portion 10a so as to be prevented from abutment against the back of the user when the user takes the sitting posture on the massager 1.

When the user turns on the power supply of the massager 1 and depresses a start button of the massage action, the operation of determining the physical constitution of the massager 1 starts (step S901).

Then, the treatment section 40, which has the massage heads (treatment applicators) 402a to 402d, starts movement to an initial position (step S902).

In association with the movement of the treatment section 40, the massage head arms 403R and 403L journaling the massage heads 402a to 402d starts slow movement from the upper portion to the lower portion. The sensor section 100 senses an impedance variance occurring between the massage head arms 403R and 403L, which function as the electrodes, as an output voltage variance (step 5903).

In the initial position, the treatment section 40 is positioned not to abut the back of the user. As such, the output voltage corresponding to the impedance occurring at the massage head arms 403R and 403L has a substantially "0" or lower value. Variations in the output voltage occur corresponding to the physical constitution of the user as the treatment section 40 descends on the back side of the user from his/her head to shoulder, back and to waist. Ordinarily, the impedance, which occurs at the massage head arms 403R and 403L takes a value smaller as the massage head arms 403R and 403L are pushed more intensively against the back surface of the user, or in other words, the shape of the back surface of the user projects greater to the side of the back rest portion, so that the current is induced to easily flow, and the output voltage is increased correspondence to that value.

Accordingly, the output voltage varies depending on, for example, the body type and body fat percentage of the user, and in an initial stage of the measurement, output voltage is simply increased (refer to FIG. 8). FIG. 8 is a graph showing measurement results of five users having physical constitutions different from one another. In the graph, the vertical axis indicates the value of the output voltage from the sensor section, and the horizontal axis indicates the number of pulses of the motor which drives the treatment section 40 representing the position in the range of from the head to waist of the respective user.

As shown in FIG. 8, in the present embodiment example, when the treatment section 40 passes across the shoulder position of the user, the output voltage takes a peak value, and thereafter reduced. Then, depending on the shape of the back, which is a to-be-massaged portion of the user and a distribution state of fat (muscles) of the user, the output voltage value transitions in the measurement range while vertically swinging.

In this event, suppose that a same massage action (pressure force) is applied to the users, regardless of the physical constitutions of the users. In this case, a case can occur where in comparison with a person having a physical constitution of a "standard type", a person having a physical constitution of a "slender type" may feel the massage action to be painful. In contrast, a case can occur where in comparison with the person having the physical constitution of the "standard type", a person having a physical constitution of a "fat type" may feel the massage action to be insufficient.

Therefore, the massager 1 according to the present embodiment, the physical constitution of the user is determined, thereby to enable a massage action suitable to the physical constitution of the user.

In more specific, the CPU 13a determines whether the position where the output value has been sensed at step S903 is within a determination range predetermined to determine the user's physical constitution (step S904). The determination range can be determined such that, for example, a portion near an upper surface of the seat portion 10b is set to be a lower end, and a portion near the lower end of the shoulder blade when a user having an average body length has taken the sitting posture on the massager 1 is set to be an upper end. Thereby, even when a person having any body length has taken the sitting posture, the lower end comes to the portion near the lower end of the body trunk. In the case of the upper end thus being set to the lower end of the shoulder blade, nonuniformity thereof is not as large as in the case of the body length. If the position is within the determination range ("YES"), then the output value is stored into a predetermined storing means, such as a memory (at step S905), and the operation proceeds to step S906. On the other hand, if the position is not within the determination range ("NO"), the operation proceeds to step S906.

Subsequently, it is determined whether the position of the respective massage head 402 has reached a lower limit position of the measurement range or a movable range for the massage action (step S906). If the position of the respective massage head 402 has not reached the lower limit ("NO"), then steps S903 to S905 are iterated, and a plurality of output values in the determination range are stored into the storing means.

If at step S906 the position of the massage head 402 has reached the lower limit ("YES"), the CPU 13a performs an averaging process of one or more output values, which have been stored in the storing means at step S907, and produces an average value A1.

Then the CPU 13a determines whether or not the average value A1 is greater than or equal to a predetermined upper limit value (which is set to an output voltage of 2.4 V, in the present embodiment examples) L1 (step S908). If A1 (= the average value) $\geq$ L1 (= upper limit value) (which corresponds to a user A shown in FIG. 8, in the present embodiment example), then it is determined that the physical constitution of the user is the fat type (step S909). Then, the data of the physical constitution of the user is stored, and the physical constitution sensing terminates (step S913).

On the other hand, in the event of A1 < L1, the CPU 13a determines whether or not the average value A1 is less or equal to a predetermined lower limit value L2 (which is an output voltage of 1.8 V, in the present embodiment example) (step S910). If A1 $\leq$ L2 (which corresponds to a user D, E shown in FIG. 8, in the present embodiment example), then it is determined that the physical constitution of the user is the slender type (step S911). Then, the data of the physical constitution of the user is stored, and the physical constitution sensing terminates (step S913). On the other hand, if A1 > L2 (which corresponds to users B and C shown in FIG. 8, in the present embodiment example), then it is determined that the physical constitution of the user is the standard type (step S912). Then, the data of the physical constitution of the user is stored, and the physical constitution sensing terminates (step S913).

As described above, while being moved at predetermined pitch, the massage head arms 403R and 403L, of which back surface side of the user staying in the sitting posture on the back rest portion 10a function as the electrodes, are measured. Thereby, as shown in FIG. 8, with output voltages being produced from the sensor section, curves corresponding to the respective physical constitutions of the user can be obtained. Accordingly, according to the present embodiment example, the physical constitution of the user can be determined from the average value of the output values in the determination range.

In the present embodiment example, two determination reference values are provided, thereby to classify the physical constitutions of the user into three grades. However, determination references may be increased, whereby physical constitutions of the user are determined even more finely, and the massage action is changed corresponding to the physical constitutions of the user. In addition, the determination range of the physical constitution may be set to a range of, for example, from a position of the shoulder blade of a user determined from a bio-signal of the user to a lower side thereof, thereby to set the range corresponding to the user.

Accordingly, according to the massager 1 of the present embodiment example, without requiring the user to set the user's own physical constitution, a massage action corresponding to the physical constitution of the user can be automatically selected. Consequently, a comfortable massage action can be performed without imposing excessive burden and uncomfortable feeling on the user.

In addition, since the massage head arms 403R and 403L are used as the electrodes, additional sensing-dedicated components are not necessary, therefore making it possible to provide low cost massagers having a simple configuration.

Further, since sensing can be performed corresponding to the number of elevation pulses for driving the motor which elevates the massage head arms, an uncomfortable site of the user can be determined with high accuracy.

[Second Embodiment Example]

(First Changing Method for Massage Pattern)

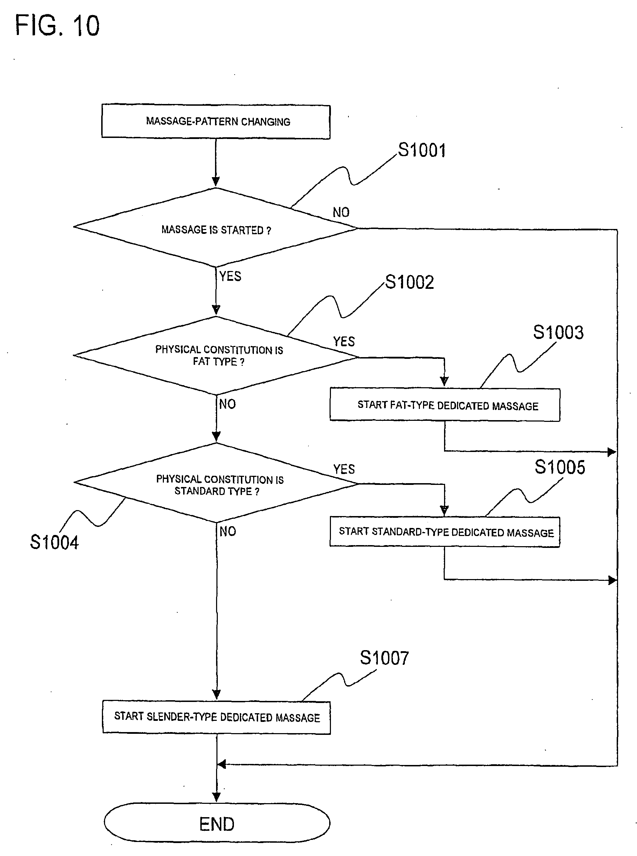

FIG. 10 is a flow diagram in the event of changing a massage pattern in the massager 1 in correspondence to the physical constitution of the user.

The massager 1 of the present embodiment example has a primary feature in that, when the user selects changing of the massage pattern, the massage pattern is changed in accordance with the respective physical constitution determined in the first embodiment example.

First, when the control section 13 determines that a massage action is started (step S1001), it is determined whether or not the physical constitution of the user is the fat type (step 51002). If the physical constitution is the fat type, then the control section 13 causes a massage action suitable to the fat type to start (step S1003) .

If the physical constitution is not the fat type, then it is determined whether or not the physical constitution of the user is the standard type (step S1004) . If the physical constitution is the standard type, the control section 13 causes a massage action suitable to the standard type to start (step S1005).

If the physical constitution is not the standard type, then it is determined that the physical constitution of the user is the slender type, and a massage action suitable to the slender type is started (step S1007).

As described above, according to the massager 1 of the present embodiment example, since the massage action is set variable in correspondence to the physical constitution of the user, the appropriate massage actions can be performed even for users having physical constitutions different in the type from one another.

Further, according to the massager 1 of the present embodiment example, without requiring the user to set the user's own physical constitution, a massage action corresponding to the physical constitution of the user can be automatically selected. Consequently, a comfortable massage action can be performed without imposing excessive burden and uncomfortable feeling on the user.

[Third Embodiment Example]

(Second Changing Method for Massage Pattern)

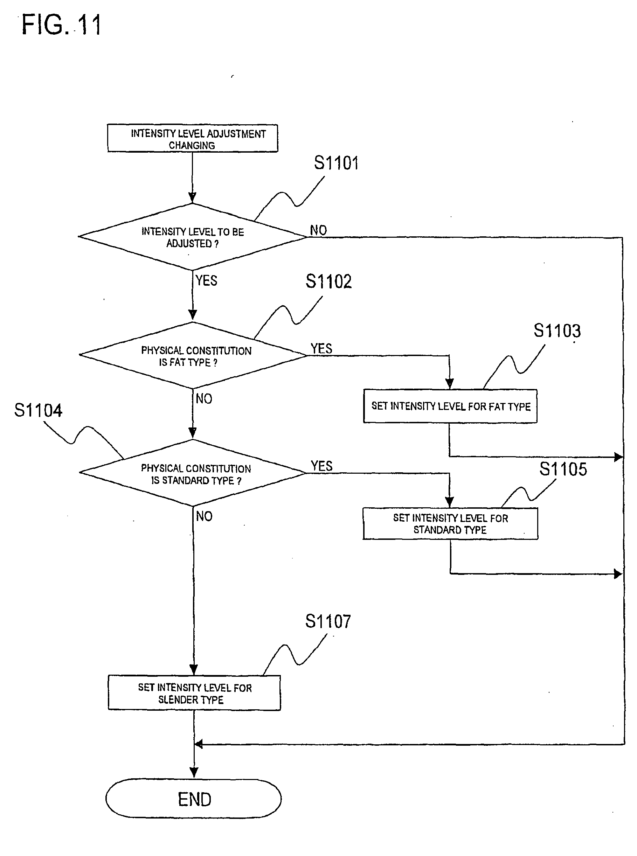

FIG. 11 is a flow diagram in the event of changing the intensity level of the pressure force of the massage action in the massager 1 in correspondence to the physical constitution of the user.

The massager 1 of the present embodiment example has a primary feature in that, when the user selects changing of the intensity level of the pressure force of the massage heads (treatment applicator) in the massage action, the intensity level of the massage heads (treatment applicator) is changed in accordance with the respective physical constitution determined in the first embodiment example.

First, when the control section 13 determines that selection is made for adjustment of an intensity level of the massage heads 402 (step S1101), it is determined whether or not the physical constitution of the user is the fat type (step S1102). If the physical constitution is the fat type, then the control section 13 sets the intensity level of the massage head 402 to an intensity level suitable to the fat type, and causes a corresponding massage action to be performed (step S1103).

If the physical constitution is not the fat type, then it is determined whether or not the physical constitution of the user is the standard type (step S1104) . If the physical constitution is the standard type, the control section 13 sets the intensity level of the massage heads 402 to an intensity level suitable to the standard type, and causes a corresponding massage action to be performed (step S1105).

If the physical constitution is not the standard type, then it is determined that the physical constitution of the user is the slender type. Accordingly, the control section 13 sets the intensity level of the massage heads 402 to an intensity level suitable to the slender type, and causes a corresponding massage action to be performed (step S1107).

As described above, according to the massager 1 of the present embodiment example, since the massage action is set variable in correspondence to the physical constitution of the user, the appropriate massage actions can be performed even for users having physical constitutions different in the type from one another.

Further, according to the massager 1 of the present embodiment example, without requiring the user to set the user's own physical constitution, a massage action corresponding to the physical constitution of the user can be automatically selected. Consequently, a comfortable massage action can be performed without imposing excessive burden and uncomfortable feeling on the user.

Thus, although the present invention has been described with reference to the embodiment and respective embodiment examples, the present invention is not limited to the respective embodiment and embodiment examples. However, the present invention may, of course, be modified and altered in various ways and be configured by optionally combining the embodiment and embodiment examples as long as permissible in accordance with scope of the invention.

**Claims**

1. A massager comprising:

   a back rest portion that supports a back side of a user;
   treatment applicators that are provided to the back rest portion and that massage the user;
   a treatment section that moves a pair of electrodes supporting the treatment applicators, in association with a massage action;
   a bio-signal sensor that senses a bio-signal of the user from an impedance variance between the pair of electrodes; and
   a control section that controls the massage action of the treatment applicators in accordance with the bio-signal, wherein
   the bio-signal sensor includes:

      an oscillation section that supplies a high frequency signal;
      the pair of electrodes that are disposed to allow the user to near or contact and that are provided to receive the high frequency signal from the oscillator section;
      a sensor section that senses the impedance variance occurring between the electrodes having received the high frequency signal when the user has neared and contacted the electrodes; and
      a signal processor section that processes a signal corresponding to the sensed impedance variance into a desired signal corresponding to the bio-signal,
      wherein the control section causes the treatment section to move and detects a signal corresponding to the bio-signal in a predetermined range, thereby to determine a physical constitution of the user.

2. A massager comprising:

   a back rest portion that supports a back side of a user;
   treatment applicators that are provided to the back rest portion and that massage the user;
   a treatment section that moves a pair of electrodes supporting the treatment applicators, in association with a massage action;
   a bio-signal sensor that senses a bio-signal of the user from an impedance variance between the pair of electrodes; and
   a control section that controls the massage action of the treatment applicators in accordance with the bio-signal, wherein
   the bio-signal sensor includes:

      an oscillation section that supplies a high frequency signal;
      the pair of electrodes that are disposed to allow the user to near or contact and that are provided to receive the high frequency signal from the oscillator section;
      a sensor section that senses the impedance variance occurring between the electrodes having received the high frequency signal when the user has neared and contacted the electrodes; and
      a signal processor section that processes a signal corresponding to the sensed impedance variance into a desired signal corresponding to the bio-signal,
      wherein the control section causes the treatment section to move and performs averaging of a plurality of signals corresponding to the bio-signal in a predetermined range, thereby to determine a physical constitution of the user.

3. A massager according to claim 2, wherein
   the control section makes a comparison between an average value obtained through the averaging of the plurality of signals and a predetermined reference value, thereby to determine the physical constitution of the user.

4. A massager according to any one of claims 1 to 3, comprising a seat portion whereon the user takes a sitting posture, wherein
the predetermined range is defined such that a portion near an upper surface of the seat portion is preset to be a lower end, and a portion near a predetermined site of a body of the user in a use state of the massager is preset to be an upper end.

5. A massager according to any one of claims 1 to 3, wherein
the predetermined range is a range below a position of a predetermined site of a user body determined from the bio-signal of the user, and is set corresponding to the user.

6. A massager according to any one of claims 1 to 5, wherein
the control section changes a pattern of massage being performed with the treatment applicators in accordance with the physical constitution of the user having been determined.

7. A massager according to any one of claims 1 to 6, wherein
the control section changes a pressure force being applied with the treatment applicators in accordance with the physical constitution of the user having been determined.

8. A massager according to any one of claims 1 to 7, wherein
the electrodes are pivotable with respect to the treatment section, and are arms each supporting a plurality of the treatment applicators; and
the plurality of treatment applicators are provided to be aligned substantially parallel to a movement direction of the treatment section in a manner to sandwich a pivoting center of the arm.

9. A massager according to any one of claims 1 to 7, wherein
the sensor section generates and supply a signal that represents a variance in a level of a reflected wave of the high frequency signal and that corresponds to the impedance variance occurring between the electrodes in correspondence to a body type of the user.

10. A massager according to any one of claims 1 to 9, wherein
the sensor section outputs reflection wave signals corresponding to real and imaginary impedance variances occurring between the electrodes in correspondence to a physical constitution of the user.

11. A massager according to any one of claims 1 to 10, wherein
the frequency of the high frequency signal is in a range of from 10 MHz to 300 MHz.

# FIG. 1

EP 1 629 817 A1

FIG. 2

402a

402b

402c

402d

1

20

10a

10

10b

FIG. 3

1

10a

FIG. 4

FIG. 5

302

304

306b

306a

305

306d

306c

303

FIG. 6

## FIG. 7

EP 1 629 817 A1

FIG. 8

DETECTED DATA OF
PHYSICAL CONSTITUTIONS

DETERMINATION RANGE

FAT TYPE

STANDARD TYPE

SLENDER TYPE

L1

L2

OUTPUT VOLTAGE (V)

4
3.5
3
2.5
2
1.5
1
0.5
0

1  4  7  10  13  16  19  22  25  28  31  34  37  40  43

HEAD - MASSAGE HEAD POSITION - WAIST

— USER A
— USER B
— USER C
— USER D
— USER E

FIG. 9

START PHYSICAL CONSTITUTION
DETERMINATION ─── S901

MOVE EACH TREATMENT APPLICATOR
TO INITIAL POSITION ─── S902

WHILE MOVING TREATMENT
APPLICATOR, MEASURE SENSOR
OUTPUT VALUE ─── S903

S904 ── WITHIN PHYSICAL CONSTITUTION
DETERMINATION RANGE ? ── NO

↓YES

S905 ── STORE OUTPUT VALUE INTO
STORING MEANS

NO ── LOWER LIMIT ? ─── S906

↓YES

PERFORM OUTPUT-VALUE
AVERAGING PROCESS ─── S907

S908 ── AVERAGE VALUE
GREATER THAN OR EQUAL TO
REFERENCE UPPER LIMIT VALUE ? ── YES

↓NO

DETERMINE PHYSICAL CONSTITUTION
TO BE FAT TYPE ─── S909

S910 ── AVERAGE VALUE
LESS OR EQUAL TO
REFERENCE LOWER LIMIT VALUE ? ── YES

↓NO

DETERMINE PHYSICAL CONSTITUTION
TO BE SLENDER TYPE

S912 ── DETERMINE PHYSICAL CONSTITUTION
TO BE STANDARD TYPE

S911

END ─── S913

21

## FIG. 10

```
        ┌─────────────────────────────┐
        │  MASSAGE-PATTERN CHANGING    │
        └─────────────────────────────┘
                      │                        S1001
                      ▼
              ╱────────────────╲           NO
             ╱  MASSAGE IS       ╲────────────────────────────┐
             ╲  STARTED ?        ╱                             │
              ╲────────────────╱                              │
                      │ YES                                    │
                      ▼                    S1002               │
              ╱────────────────╲       YES          S1003      │
             ╱ PHYSICAL          ╲──────────┐                  │
             ╲ CONSTITUTION IS    ╱         ▼                  │
             ╲ FAT TYPE ?        ╱     ┌──────────────────────┐│
              ╲────────────────╱      │ START FAT-TYPE        ││
                      │ NO            │ DEDICATED MASSAGE     ││
                      ▼               └──────────────────────┘│
              ╱────────────────╲       YES     S1005          │
             ╱ PHYSICAL          ╲──────────┐                 │
             ╲ CONSTITUTION IS    ╱         ▼                 │
             ╲ STANDARD TYPE ?   ╱     ┌──────────────────────┐
        S1004 ╲────────────────╱      │ START STANDARD-TYPE   │
                      │ NO            │ DEDICATED MASSAGE     │
                      │               └──────────────────────┘
                      │                          S1007
                      ▼
        ┌─────────────────────────────────────┐
        │ START SLENDER-TYPE DEDICATED MASSAGE │
        └─────────────────────────────────────┘
                      │
                      ▼
                 ╭─────────╮
                 │   END   │
                 ╰─────────╯
```

# FIG. 11

INTENSITY LEVEL ADJUSTMENT
CHANGING

S1101

INTENSITY LEVEL TO BE
ADJUSTED ?

NO

YES

S1102

PHYSICAL CONSTITUTION
IS FAT TYPE ?

YES

S1103

NO

SET INTENSITY LEVEL FOR FAT TYPE

S1104

PHYSICAL CONSTITUTION
IS STANDARD TYPE ?

YES

S1105

NO

SET INTENSITY LEVEL FOR
STANDARD TYPE

S1107

SET INTENSITY LEVEL FOR
SLENDER TYPE

END

23

**European Patent Office**

# EUROPEAN SEARCH REPORT

Application Number

EP 05 01 8707

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/37729 A (OMRON CORPORATION; KASAI, EIJI; FUKUI, RYO) 31 May 2001 (2001-05-31) * figures 1-6 * | 1-11 | A61H1/00 A61H37/00 A61B5/053 |
| A | PATENT ABSTRACTS OF JAPAN vol. 2000, no. 15, 6 April 2001 (2001-04-06) -& JP 2000 342644 A (YA MAN LTD), 12 December 2000 (2000-12-12) * abstract; figures * | 1-11 | |
| A | EP 1 230 904 A (SANYO ELECTRIC CO., LTD) 14 August 2002 (2002-08-14) * the whole document * | 1-11 | |
| A | US 2002/123704 A1 (HORI KUNIHIKO ET AL) 5 September 2002 (2002-09-05) * the whole document * | 1-11 | |
| A | EP 0 989 671 A (OMRON HEALTHCARE CO., LTD) 29 March 2000 (2000-03-29) * the whole document * | 1-11 | TECHNICAL FIELDS SEARCHED (IPC) A61H A61B |

The present search report has been drawn up for all claims

| Place of search | Date of completion of the search | Examiner |
|---|---|---|
| Munich | 23 November 2005 | Elmar Fischer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 01 8707

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2005

| Patent document cited in search report | | Publication date | Patent family member(s) | | Publication date |
|---|---|---|---|---|---|
| WO 0137729 | A | 31-05-2001 | AU<br>CN<br>JP | 1416301 A<br>1391450 A<br>3575463 B2 | 04-06-2001<br>15-01-2003<br>13-10-2004 |
| JP 2000342644 | A | 12-12-2000 | NONE | | |
| EP 1230904 | A | 14-08-2002 | CN<br>JP<br>TW<br>US | 1368040 A<br>2002233558 A<br>508239 B<br>2002138023 A1 | 11-09-2002<br>20-08-2002<br>01-11-2002<br>26-09-2002 |
| US 2002123704 | A1 | 05-09-2002 | TW | 510789 B | 21-11-2002 |
| EP 0989671 | A | 29-03-2000 | JP<br>JP<br>US | 3484355 B2<br>2000098048 A<br>6545614 B1 | 06-01-2004<br>07-04-2000<br>08-04-2003 |

EPO FORM P0459

For more details about this annex : see Official Journal of the European Patent Office, No. 12/82